(19) Europäisches Patentamt
European Patent Office
Office européen des brevets

(11) Veröffentlichungsnummer : **0 086 450**
**B1**

(12) # EUROPÄISCHE PATENTSCHRIFT

(45) Veröffentlichungstag der Patentschrift : **28.05.86**

(51) Int. Cl.[4] : **C 07 D231/12**, C 07 D207/22, A 61 K 31/415

(21) Anmeldenummer : **83101224.0**

(22) Anmeldetag : **09.02.83**

(54) **Substituierte Phenylpyrazolderivate, Verfahren zu ihrer Herstellung, Arzneimittel auf Basis dieser Verbindungen, sowie deren Verwendung.**

(30) Priorität : **13.02.82 DE 3205187**

(43) Veröffentlichungstag der Anmeldung : **24.08.83 Patentblatt 83/34**

(45) Bekanntmachung des Hinweises auf die Patenterteilung : **28.05.86 Patentblatt 86/22**

(84) Benannte Vertragsstaaten : **AT BE CH DE FR GB IT LI NL**

(56) Entgegenhaltungen :
**US-H- 535 437**
**Patent Abstracts of Japan Band 6, Nr. 110, 22. Juni 1982**

(73) Patentinhaber : **HOECHST AKTIENGESELLSCHAFT**
**Postfach 80 03 20**
**D-6230 Frankfurt am Main 80 (DE)**

(72) Erfinder : **Kunstmann, Rudolf, Dr.**
**Auf der Ahl 37**
**D-6200 Wiesbaden (DE)**
Erfinder : **Bickel, Martin, Dr.**
**Beethovenstrasse 40**
**D-6000 Frankfurt am Main (DE)**

**EP 0 086 450 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft substituierte Phenylpyrazolderivate der allgemeinen Formel I

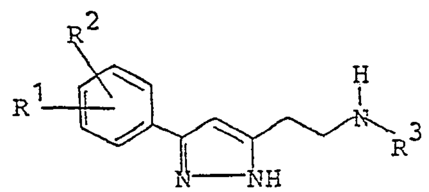

(I)

und ihre Salze mit physiologisch verträglichen Säuren, die aufgrund ihrer pharmakologischen Eigenschaften als Arzneimittel verwendet werden können.

In der Formel I bedeuten :

$R^1$ und $R^2$, die gleich oder verschieden sein können, Wasserstoff, Halogen, Alkyl oder Alkoxy mit 1 bis 4 Kohlenstoffatomen, Nitro oder Amino,

$R^3$ Wasserstoff, einen gesättigten oder ungesättigten, geradkettigen oder verzweigten aliphatischen Kohlenwasserstoffrest mit 1 bis 8 Kohlenstoffatomen, oder einen Cycloalkylrest mit 5 bis 8 Kohlenstoffatomen, wobei der Kohlenwasserstoffrest oder Cylcloalkylrest seinerseits mit einem Phenylrest oder einem mit Halogen, Nitro, Amino oder einem Alkyl- oder Alkoxyrest mit 1 bis 4 Kohlenstoffatomen mono- oder disubstituierten Phenylrest substituiert sein kann.

Bevorzugt sind solche Verbindungen der Formel I, worin $R^1$ und $R^2$ gleich oder verschieden sein können und Wasserstoff, Chlor, Fluor, Methyl oder Methoxy bedeuten, und $R^3$ Wasserstoff oder eine Alkylkette mit 1 bis 4 Kohlenstoffatomen darstellt, die ihrerseits mit einem Phenylrest oder einem mit Chlor, Fluor, Methyl, Methoxy, Nitro oder Aminorest mono- oder disubstituierten Phenylrest substituiert sein kann.

Besonders bevorzugt sind solche Verbindungen der Formel I, worin $R^1$ Wasserstoff und $R^2$ Chlor, Fluor, Methyl oder Methoxy bedeuten, oder worin $R^1$ und $R^2$ beide Methoxy bedeuten, und $R^3$ Wasserstoff oder eine Alkylkette mit 1 bis 4 Kohlenstoffen darstellt, die ihrerseits mit einem Phenylrest oder einem mit Chlor, Fluor, Methyl, Methoxy, mono- oder disubstituierten Phenylrest substituiert sein kann.

Die erfindungsgemäßen Verbindungen der Formel I sind neu. Sie hemmen im Tiermodell die durch Pentagastrin stimulierte Magensäuresekretion und stellen damit Arzneimittel für die Prophylaxe und die Therapie von Magenulcera dar. Darüberhinaus dienen sie als Zwischenprodukte für die Synthese neuer Arzneimittel.

Gegenstand der Erfindung ist weiterhin ein Verfahren zur Herstellung von Verbindungen der Formel I, dadurch gekennzeichnet, daß man ein Aminopyrrolin der Formel II

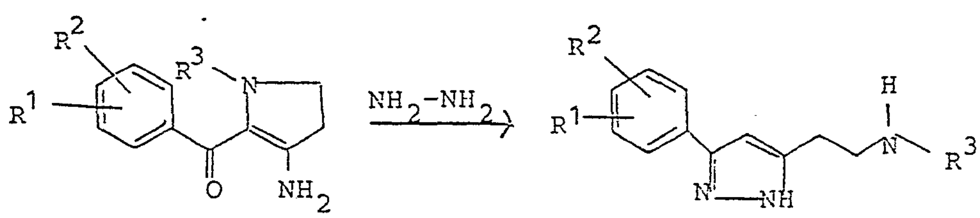


(II) (I)

mit Hydrazin zu einer Verbindung der Formel I umsetzt, wobei $R^1$, $R^2$ und $R^3$ die vorstehend zu Formel I angegebenen Bedeutungen haben, und gegebenenfalls eine Verbindung der Formel I, in der $R^3$ Benzyl bedeutet, durch Abspalten der Benzylgruppe in eine Verbindung der Formel I überführt, in der $R^3$ Wasserstoff bedeutet.

Im allgemeinen werden 1,3-Dicarbonylverbindungen oder deren Derivate, die β-Aminovinylketone, mit Hydrazin glatt zu den entsprechenden Pyrazolderivaten cyclisiert (A. N. Kost, I. I. Grandberg, Advances in Heterocyclic Chemistry, Edited by A. R. Katritzky, A. J. Boulton, Bd. 6, S. 358 ff, Academic Press, New York, London 1966). Im vorliegenden Fall würde man dementsprechend Pyrrolo-[3,2-c] pyrazole (IIa) erwarten. Es war deshalb überraschend, daß bei der Reaktion von II mit Hydrazin Verbindungen I entstehen, die sich von den ursprünglich erwarteten durch einen zusätzlichen Reduktionsschritt unterscheiden, bei dem sich der Pyrrolinring des Ausgangsmaterials öffnet.

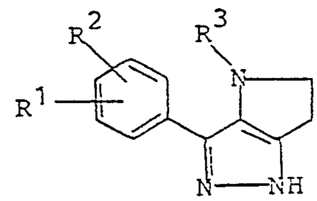

IIa

Die Ausgangsstoffe der Formel II sind neu. Sie werden auf folgenden Wegen synthetisiert. Aus substituierten Phenacylhalogeniden III stellt man durch Umsetzung mit substituierten 3-Aminopropionsäurenitrilen IV die Aminoketone V her, worin $R^1$, $R^2$ und $R^3$ die zu Formel I genannten Bedeutungen haben und X Halogen, insbesondere Chlor oder Brom, bedeutet.

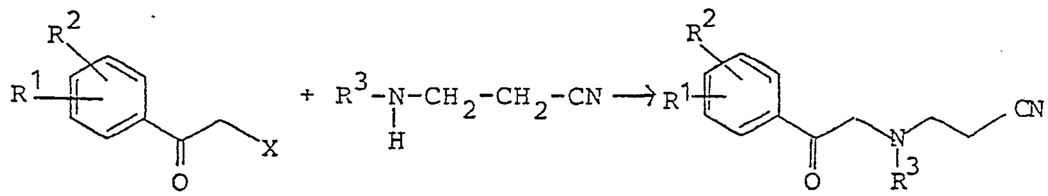

(III) (IV) (V)

Die Verbindungen IV enthält man durch Michaeladdition von Aminen ($R^3$—$NH_2$) an äquimolare Mengen Acrylnitril in Äthanol.

Die Umsetzung zu Verbindungen V führt man zweckmäßig in einem inerten Lösungsmittel wie Äther, Aceton oder einem chlorierten Kohlenwasserstoff wie Methylenchlorid durch, wobei die Reaktions-Temperatur zwischen —30 °C und + 80 °C liegen kann.

Der bei der Reaktion von III mit IV gebildete Halogenwasserstoff wird zweckmäßig durch eine geeignete Base abgefangen. Besonders gut eignen sich hierfür tertiäre Amine, die keine Quaternierung mit III eingehen können. Geeignete Amine sind somit beispielsweise 1,4-Diazabicyclo[2,2,2]oktan, 1,5-Diazabicyclo[4.3.0]non-5-en oder N,N-Diisopropyl-äthylamin. Am einfachsten arbeitet man jedoch mit einem doppelten Überschuß an eingesetztem Amin IV. Sofern $R^3$ Wasserstoff darstellt, wird bevorzugt ein Gemisch aus 3-Aminopropionsäurenitril (IV, $R^3$ = H) und einem oben genannten Säurefänger, z. B. in einem oben genannten Lösungsmittel, vorgelegt und das Phenacylhalogenid gelöst im selben Lösungsmittel bei Temperaturen unterhalb von 5-8 °C zugetropft.

Die Verbindungen V können für die anschließende Cyclisierung zu den Verbindungen II auch als Rohprodukte eingesetzt werden, so daß eine Reinigung durch Kristallisation oder Destillation entfallen kann.

Die Cyclisierung von (V) zu (II) wird im allgemeinen im basischen Medium durchgeführt. Dazu wird eine Verbindung (V) in einem geeigneten Alkohol wie Methanol, Äthanol, Isopropanol oder tert.-Butylalkohol mit einem entsprechenden Alkalialkoholat, vorzugsweise Natriumalkoholat, behandelt. Die Reaktionstemperatur liegt zwischen —20 °C und der Rückflußtemperatur der Reaktionsmischung. Nach Ende der Reaktion kann das Produkt durch Zugabe von Wasser isoliert werden.

Die Cyclisierung kann auch unter sauren Bedingungen durchgeführt werden. Dabei hat sich der Einsatz einer Lewis-Säure wie Aluminiumchlorid oder Bortrifluorid bewährt. So wird beispielsweise eine Verbindung V unter Kühlung in Bortrifluoridätherat eingetragen und die Reaktionslösung wird je nach Substituenten bei einer Temperatur zwischen —10 °C und der Rückflußtemperatur des Reaktionsgemisches bis zur Beendigung der Umsetzung gehalten. Durch Zugabe von Wasser kristallisieren in der Kälte die Verbindungen VI aus, oder sie können durch Extraktion mittels eines organischen Lösungsmittels abgetrennt werden.

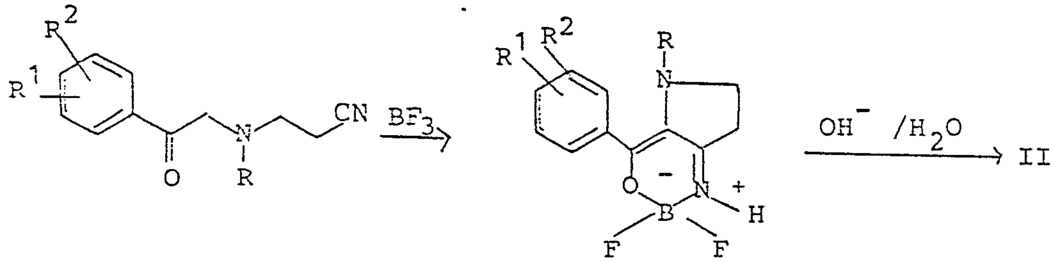


(V) (VI)

Durch Behandlung der erhaltenen Verbindung VI mit wässrigen Alkalien wie z. B. Natronlauge,

Ammoniak- oder Bicarbonatlösung wird diese leicht zu einer Verbindung II hydrolytisch gespalten.

Dieses Verfahren der sauren Cyclisierung hat sich besonders bewährt, wenn $R^1$ und $R^2$ Methoxysubstituenten darstellen.

Zur Umsetzung der Verbindungen II zu I werden diese mit Hydrazinhydrat behandelt. Die Anwendung von Rückflußtemperaturen in Hydrazinhydrat oder in alkoholischen Lösungen ist vorteilhaft. Als Alkohole kommen sowohl primäre Alkohole wie Methanol, Äthanol, sekundäre Alkohole wie Isopropanol als auch tertiäre Alkohole wie beispielsweise tertiär-Butanol in Betracht.

Nach beendeter Umsetzung wird die Reaktionsmischung eingeengt und die Verbindung I entsprechend ihres basischen Charakters isoliert, indem sie mit einer Mineralsäure in ein wasserlösliches Salz übergeführt, die wäßrige Salzlösung zur Entfernung neutraler Verunreinigungen mit einem mit Wasser nicht mischbaren Lösungsmittel extrahiert und danach die Base durch alkalisch stellen der Wasserphase wieder in Freiheit gesetzt wird. Es ist auch möglich, die Verbindung I durch Zugabe von Wasser auszufällen oder mittels eines organischen, mit Wasser nicht mischbaren Lösungsmittel zu extrahieren.

Die gegebenenfalls durchzuführende Abspaltung einer Benzylgruppe kann wie folgt durchgeführt werden.

In erster Linie hat sich die hydrogenolytische Spaltung in Gegenwart von Raney-Nickel im zweckmäßig sauren Medium bewährt. Daneben kommen noch andere Methoden in Betracht, wie z. B. die Spaltung mit elementarem Natrium in flüssigem Ammoniak oder die Behandlung mit Tetralin oder einem anderen leicht oxidierbaren und Wasserstoff abspaltenden Kohlenwasserstoff in Gegenwart eines Edelmetallkatalysators wie z. B. Palladium auf Kohle bei erhöhter Temperatur, insbesondere bei 110-180 °C.

Die erfindungsgemäßen Verbindungen haben wertvolle pharmakologische Eigenschaften. So hemmen sie in Tierversuchen die Magensäuresekretion. Sie eignen sich deshalb als Arzneimittel zur Prophylaxe und Therapie von Magenulcera.

Die neuen Verbindungen der Formel I können entweder allein oder mit physiologisch verträglichen Hilfs- oder Trägerstoffen vermischt als Arzneimittel angewandt werden. Für eine orale Anwendungsform werden die Verbindungen der Formel I mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige alkoholische oder ölige Lösungen. Als inerte Träger können z. B. Magnesiumcarbonat, Milchzucker oder Maisstärke verwendet werden. Für Tabletten kann die Zubereitung als Trocken- oder Feuchtgranulat erfolgen. Als ölige Trägerstoffe oder Lösungsmittel kommen pflanzliche oder mineralische Öle in Betracht, wie z. B. Sonnenblumenöl, Olivenöl oder Paraffinöl.

Zur intravenösen Applikation werden die aktiven Verbindungen oder deren Salze mit physiologisch verträglichen organischen oder anorganischen Säuren in dafür üblichen Substanzen gelöst. Als organische Säuren seien beispielsweise genannt : Essigsäure, Propionsäure, Milchsäure, Malonsäure, Bernsteinsäure, Maleinsäure, Fumarsäure, Zitronensäure, Äpfelsäure, Weinsäure, Benzoesäure, Oxäthansulfonsäure, Acetursäure (Acetylaminoessigsäure), Äthylendiamintetraessigsäure, Embonsäure (1,1'-Methylen-bis-(2-hydroxy-3-naphthoesäure) sowie saure Gruppen enthaltende synthetische Harze. Als anorganische Säuren kommen beispielsweise in Betracht : Halogenwasserstoffsäure, wie Chlorwasserstoffsäure oder Bromwasserstoffsäure, Schwefelsäure, Phosphorsäure und Amidosulfonsäure.

Als Lösungsmittel für die intravenöse Applikation kommen in Betracht Wasser oder physiologische Kochsalzlösung, die mit weiteren Zusätzen wie Alkoholen (z. B. Propandiol, Glycerin) oder Zuckerlösungen (z. B. Glukose- oder Mannitlösungen) versehen sein können.

Beispiel 1

5-(2-Methylaminoäthyl)-3-phenyl-pyrazol

a) N-Methyl-N-(2-phenyl-2-oxo-äthyl)-3-aminopropionsäurenitril

In 100 ml Aceton löst man 0,1 Mol Phenacylbromid und tropft dazu bei 0 °C 0,2 Mol 3-Methylaminopropionsäurenitril, gelöst in 50 ml Aceton. Man rührt 15 Stunden bei Raumtemperatur, filtriert die Lösung vom ungelösten Ammoniumsalz ab und engt sie ein. Das Rohprodukt ist für die weitere Umsetzung rein genug.

b) 3-Amino-2-benzoyl-1-methyl-4,5-dihydro-pyrrol

0,1 Mol N-Methyl-N-(2-phenyl-2-oxo-äthyl)-3-aminopropionsäurenitril werden in 200 ml Methanol gelöst. Nach Zugabe von 0,3 Mol Natriummethylat wird die Mischung zwei Stunden am Rückfluß gehalten. Anschließend gießt man die Reaktionsmischung auf 800 ml Eis/Wasser-Gemisch, extrahiert mit Methylenchlorid, trocknet die organische Phase und engt sie ein. Das zurückbleibende Öl kristallisiert langsam (F : 115-117 °C) und ist für die weitere Umsetzung rein genug.

c) 5-(2-Methylaminoäthyl-3-phenyl-pyrazol

0,03 Mol 3-Amino-2-benzoy-1-methyl-4,5-dihydropyrrol werden in 50 ml Äthanol nach Zusatz von 8 ml Hydrazinhydrat vier Stunden am Rückfluß erhitzt. Danach gießt man die Reaktionsmischung auf 200 ml Wasser und extrahiert sie mit Essigester. Nach dem Trocknen und Einengen der organischen Phase hinterbleibt ein öliges Rohprodukt, das in das oxalsaure Salz überführt wird.
F. : 187 °C (Oxalat).

Beispiel 2

5-(2-Benzylaminoäthyl)-3-phenyl-pyrazol

a) N-Benzyl-N-(2-phenyl-2-oxo-äthyl)-3-aminopropionsäurenitril

Entsprechend Beispiel 1a aus Phenacylbromid und 3-Benzylaminopropionsäurenitril. Rohprodukt weiter umgesetzt.

b) 3-Amino-2-benzoyl-1-benzyl-4,5-dihydro-pyrrol

Entsprechend Beispiel 1b aus N-Benzyl-N-(2-phenyl-2-oxo-äthyl)-3-aminopropionsäurenitril.
F. : 149-150 °C (Diäthyläther)

c) 5-(2-Benzylaminoäthyl)-3-phenyl-pyrazol

Entsprechend Beispiel 1c aus 3-Amino-2-benzoyl-1-benzyl-4,5-dihydro-pyrrol und Hydrazin in Methanol
F. : 109 °C (Freie Base) 213-215 °C (Hydrochlorid).

Beispiel 3

5-(2-Benzylaminoäthyl)-3-(3,4-dimethoxyphenyl)-pyrazol

a) N-Benzyl-N-[2-(3,4-dimethoxyphenyl)-2-oxo-äthyl]-3-aminopropionsäurenitril

Entsprechend Beispiel 1a aus 3,4-Dimethoxyphenacylbromid und 3-Benzylaminopropionsäurenitril.
F. : 79-82 °C (Diäthyläther)

b) 1-Benzyl-7-(3,4-dimethoxyphenyl)-5,5-difluor-2,3-dihydro-6-oxa-4-azonia-5-borato-indol

0,1 Mol N-Benzyl-N-[2-(3,4-dimethoxyphenyl)-2-oxoäthyl]-3-amino-propionsäurenitril werden in 100 ml Bortrifluoridätherat 16 Stunden bei 90-95 °C geführt. Man gießt die Reaktionsmischung anschließend auf Eis, stellt mit wäßriger Ammoniaklösung neutral und extrahiert mit Essigester. Nach dem Trocknen und Einengen der organischen Phase hinterbleibt ein Öl, das aus Isopropanol kristallisiert wird.
F. : 175-176 °C.

c) 3-Amino-1-benzyl-2-(3,4-dimethoxybenzoyl)-4,5-dihydropyrrol

0,05 Mol der nach Beispiel 3b hergestellten Verbindung werden in 100 ml conc. wäßriger Natriumhydroxidlösung suspendiert und 24 Stunden bei Raumtemperatur gerührt. Man saugt den Niederschlag ab, wäscht neutral und trocknet den Filterkuchen.
F. : 134-136 °C.

d) 5-(2-Benzylaminoäthyl)-3-(3,4-dimethoxyphenyl)-pyrazol

Aus 3-Amino-1-benzyl-2-(3,4-dimethoxybenzoyl)-4,5-dihydropyrrol durch Behandlung mit Hydrazin in t-Butanol entsprechend Beispiel 1c.

Beispiel 4

5-[2-(2-Chlorbenzylamino)-äthyl]-3-phenyl-pyrazol

a) N-(2-Chlorbenzyl)-N-(2-phenyl-2-oxo-äthyl)-3-aminopropionsäurenitril.

Aus 1 Mol Phenacylbromid und 1 Mol 3-(2-Chlorbenzylamino)-propionsäurenitril entsprechend Beispiel 1a mit 1 Mol N,N-Diisopropyl-äthylamin als Säurefänger. Das Rohprodukt wurde weiter umgesetzt.

b) 3-Amino-2-benzoyl-1-(2-chlorbenzyl)-4,5-dihydropyrrol.

Aus N-(2-Chlorbenzyl)-N-(2-phenyl-2-oxo-äthyl)-3-amino-propionsäurenitril durch Behandlung mit Methylat analog Beispiel 1b.
F. : 151 °C.

c) 5-[2-(2-Chlorbenzylamino)-äthyl]-3-phenyl-pyrazol.

Aus 3-Amino-2-benzoyl-1-(2-chlorbenzyl)-4,5-dihydropyrrol durch Behandlung mit Hydrazin in Methanol entsprechend Beispiel 1c.
F. : 206-207 °C (HCl).

Beispiel 5

5-[2-(4-Methylbenzylamino)-äthyl]-3-phenyl-pyrazol

a) N-(4-Methylbenzyl)-N-(2-phenyl-2-oxo-äthyl)-3-aminopropionsäurenitril

Aus Phenacylbromid und 3-(4-Methylbenzylamino)-propionsäurenitril entsprechend Beispiel 1a.

b) 3-Amino-2-benzoyl-1-(4-methylbenzyl)-4,5-dihydropyrrol.

Aus N-(4-Methylbenzyl)-N-(2-phenyl-2-oxo-äthyl)-3-amino-propionsäurenitril durch Behandlung mit Methylat analog Beispiel 1b.

c) 5-[2-(4-Methylbenzylamino)-äthyl]-3-phenyl-pyrazol.

Aus 3-Amino-2-benzoyl-1-(4-methylbenzyl)-4,5-dihydropyrrol durch Behandlung mit Hydrazin in Isopropanol entsprechend Beispiel 1c.

Beispiel 6

5-[2-(3-Methoxybenzylamino)-äthyl]-3-phenyl-pyrazol

a) N-(3-Methoxybenzyl)-N-(2-phenyl-2-oxo-äthyl)-3-aminopropionsäurenitril.

Aus 1 Mol Phenacylbromid und 1 Mol 3-(3-Methoxybenzylamino)-propionsäurenitril entsprechend Beispiel 1a, mit 1 Mol N,N-Diisopropyläthylamin als Säurefänger.

b) 3-Amino-2-benzoyl-1-(3-methoxybenzyl)-4,5-dihydropyrazol.

Aus N-(3-Methoxybenzyl)-N-(2-phenyl-2-oxo-äthyl)-3-aminopropionsäurenitril durch Behandlung mit Methylat analog Beispiel 1b.

c) 5-[2-(3-Methoxybenzylamino)-äthyl]-3-phenylpyrazol

Aus 3-Amino-2-benzoyl-1-(3-methoxybenzyl)-4,5-dihydropyrrol durch Behandlung mit Hydrazin in Methanol entsprechend Beispiel 1c.
F. : 195 °C (Oxalat).

Beispiel 7

5-[2-(3,4-Dimethoxybenzylamino)-äthyl]-3-phenylpyrazol

a) N-(3,4-Dimethoxybenzyl)-N-(2-phenyl-2-oxo-äthyl)-3-amino-propionsäurenitril.

Aus Phenacylbromid und 3-(3,4-Dimethoxybenzylamino)-propionsäurenitril entsprechend Beispiel 1a.

b) 3-Amino-2-benzoyl-1-(3,4-dimethoxybenzyl)-4,5-dihydropyrrol.

Aus N-(3,4-Dimethoxyphenyl)-N-(2-phenyl-2-oxo-äthyl)-3-amino-propionsäurenitril durch Behandlung mit Methylat analog Beispiel 1b.

c) 5-[2-(3,4-Dimethoxybenzylamino)-äthyl]-3-phenylpyrazol

Aus 3-Amino-2-benzoyl-1-(3,4-dimethoxybenzyl)-4,5-dihydro-pyrrol durch Behandlung mit Hydrazin in Äthanol entsprechend Beispiel 1c.

Beispiel 8

5-[2-(2-Phenyläthylamino)-äthyl]-3-phenyl-pyrazol

a) N-(2-Phenyläthyl)-N-(2-phenyl-2-oxo-äthyl)-3-aminopropionsäurenitril.

Aus Phenacylbromid und 3-(2-Phenyläthylamino)-propionsäurenitril entsprechend Beispiel 1a.

b) 3-Amino-2-benzoyl-1-(2-Phenyläthyl)-4,5-dihydropyrrol

Aus N-(2-Phenyläthyl)-N-(2-phenyl-2-oxo-äthyl)-3-amino-propionsäurenitril durch Behandlung mit Methylat analog Beispiel 1b.

c) 5-[2-(2-Phenyläthylamino)-äthyl]-3-phenyl-pyrazol

Aus 3-Amino-2-benzoyl-1-(2-phenyläthyl)-4,5-dihydropyrrol durch Behandlung mit Hydrazin in Äthanol entsprechend Beispiel 1c.

Beispiel 9

5-[2-(3-Phenylpropylamino)-äthyl]-3-phenyl-pyrazol

a) N-(3-Phenylpropyl)-N-(2-phenyl-2-oxo-äthyl)-3-amino-propionsäurenitril

Aus 1 Mol Phenacylbromid und 1 Mol 3-(3-Phenylpropylamino)-propionsäurenitril entsprechend Beispiel 1a, mit 1 Mol N,N-Diisopropyl-äthylamin als Säurefänger.

b) 3-Amino-2-benzoyl-1-(3-phenylpropyl)-4,5-dihydropyrrol

Aus N-(3-Phenylpropyl)-N-(2-phenyl-2-oxo-äthyl)-3-amino-propionsäurenitril durch Behandlung mit Methylat analog Beispiel 1b.

c) 5-[2-(3-Phenylpropylamino)-äthyl]-3-phenyl-pyrazol

Aus 3-Amino-2-benzoyl-1-(3-phenylpropyl)-4,5-dihydropyrrol durch Behandlung mit Hydrazin in Isopropanol entsprechend Beispiel 1c.

Beispiel 10

5-[2-Benzylamino-äthyl]-3-(4-chlorphenyl)-pyrazol

a) N-Benzyl-N-(2-(4-chlorphenyl)-2-oxo-äthyl)-3-amino-propionsäurenitril

Aus 4-Chlorphenacylbromid und 3-(Benzylamino)-propionsäurenitril entsprechend Beispiel 1a.

b) 3-Amino-2-(4-chlorbenzoyl)-1-benzyl-4,5-dihydropyrrol

Aus N-(Benzyl)-N-(2-(4-chlorphenyl)-2-oxo-äthyl)-3-amino-propionsäurenitril durch Behandlung mit Methylat analog Beispiel 1b.

c) 5-[2-Benzylamino-äthyl]-3-(4-chlorphenyl)-pyrazol

Aus 3-Amino-2-(4-chlorbenzoyl)-1-benzyl-4,5-dihydropyrrol durch Behandlung mit Hydrazin in Äthanol entsprechend Beispiel 1c.
F. : 182-184 °C (HCl).

Beispiel 11

5-[2-(2-Chlorbenzylamino)-äthyl-3-(4-chlorphenyl)-pyrazol

a) N-(2-Chlorbenzyl)-N-[2-(4-chlorphenyl)-2-oxo-äthyl]-3-amino-propionsäurenitril

Aus 4-Chlorphenacylbromid und 3-(2-Chlorbenzylamino)-propionsäurenitril entsprechend Beispiel 1a.

b) 3-Amino-2-(4-chlorbenzoyl)-1-(2-chlorbenzyl)-4,5-dihydro-pyrrol.

Aus N-(2-Chlorbenzyl)-N-[2-(4-chlorphenyl)-2-oxoäthyl]-3-amino-propionsäurenitril durch Behandlung mit Methylat analog Beispiel 1b.

c) 5-[2-(2-Chlorbenzylamino)-äthyl]-3-(4-chlorphenyl)-pyrazol.

Aus 3-Amino-2-(4-chlorbenzoyl)-1-(2-chlorbenzyl)-4,5-dihydro-pyrrol durch Behandlung mit Hydrazin in Alkohol entsprechend Beispiel 1c.
F.: 241 °C (2HCl).

Beispiel 12

5-[2-Methylamino-äthyl]-3-(4-chlorphenyl)-pyrazol

a) N-Methyl-N-[2-(4-chlorphenyl)-2-oxo-äthyl]-3-amino-propionsäurenitril.

Aus 4-Chlorphenacylbromid und 3-Methylamino-propionsäurenitril entsprechend Beispiel 1a.

b) 3-Amino-2-(4-chlorbenzoyl)-1-methyl-4,5-dihydropyrrol.

Aus N-Methyl-N-[2-(4-chlorphenyl)-2-oxo-äthyl]-3-amino-propionsäurenitril durch Behandlung mit Methylat analog Beispiel 1b.
F.: 143 °C.

c) 5-[2-Methylamino-äthyl]-3-(4-chlorphenyl)-pyrazol

Aus 3-Amino-2-(4-chlorbenzoyl)1-methyl-4,5-dihydropyrrol durch Behandlung mit Hydrazin in Alkohol entsprechend Beispiel 1c.
F.: 118 °C.

Beispiel 13

5-[2-(4-Chlorbenzylamino)-äthyl]-3-phenyl-pyrazol

a) N-(4-Chlorbenzyl)-N-[2-phenyl-2-oxo-äthyl]-3-amino-propionsäurenitril

Aus Phenacylbromid und 3-(4-Chlorbenzylamino)-propionsäurenitril entsprechend Beispiel 1a.

b) 3-Amino-2-benzoyl-1-(4-chlorbenzyl)-4,5-dihydropyrrol.

Aus N-(4-Chlorbenzyl)-N-[2-phenyl-2-oxo-äthyl]-3-amino-propionsäurenitril durch Behandlung mit Methylat analog Beispiel 1b.
F.: 175 °C

c) 5-[2-(4-Chlorbenzylamino)-äthyl]-3-phenyl-pyrazol

Aus 3-Amino-2-benzoyl-1-(4-chlorbenzyl)-4,5-dihydropyrrol durch Behandlung mit Hydrazin in Alkohol entsprechend Beispiel 1c.
F.: 156 °C (2HCl).

Beispiel 14

5-[2-(2-Chlorbenzylamino)-äthyl]-3-phenyl-pyrazol

a) N-(2-Chlorbenzyl-N-[2-phenyl-2-oxo-äthyl]-3-amino-propionsäurenitril.

Aus Phenacylbromid und 3-(2-chlorbenzylamino)-propionsäurenitril entsprechend Beispiel 1a.

b) 3-Amino-2-benzoyl-1-(2-chlorbenzyl)-4,5-dihydropyrrol.

Aus N-(2-Chlorbenzyl)-N-[2-phenyl-2-oxo-äthyl]-3-amino-propionsäurenitril durch Behandlung mit Methylat analog Beispiel 1b.

F. : 151 °C

c) 5-[2-(2-Chlorbenzylamino)-äthyl]-3-phenyl-pyrazol.

Aus 3-Amino-2-benzoyl-1-(2-chlorbenzyl)-4,5-dihydropyrrol durch Behandlung mit Hydrazin in Alkohol entsprechend Beispiel 1c.

Beispiel 15

5-(2-Aminoäthyl)3-phenyl-pyrazol

Aus 5-(2-Benzylaminoäthyl)-3-phenyl-pyrazol durch katalytische Hydrierung mit Raney-Nickel aus Katalysator in Methanol bei 50 °C und 50 atü während 5 Stunden Reaktionszeit. Nach dem Abtrennen des Katalysators hinterbleibt ein aus Essigester kristallisierbares Öl.

F. : 118-120 °C.

**Patentansprüche** (für die Vestragsstaaten : BE, CH, DE, FR, GB, IT, LI, NL)

1. Substituierte Phenylpyrazolderivate der allgemeinen Formel I

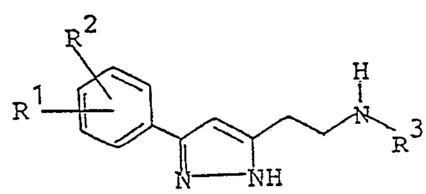

(I)

und ihre Salze mit physiologisch verträglichen Säuren, worin .

$R^1$ und $R^2$ gleich oder verschieden sein können und Wasserstoff, Halogen, Alkyl oder Alkoxy mit 1 bis 4 Kohlenstoffatomen, Nitro oder Amino,

$R^3$ Wasserstoff, einen gesättigten oder ungesättigten, geradkettigen oder verzweigten aliphatischen Kohlenwasserstoffrest mit 1 bis 8 Kohlenstoffatomen, oder einen Cycloalkylrest mit 5 bis 8 Kohlenstoffatomen, wobei der Kohlenwasserstoffrest oder Cycloalkylrest seinerseits mit einem Phenylrest oder einem mit Halogen, Nitro, Amino oder einem Alkyl- oder Alkoxyrest mit 1 bis 4 Kohlenstoffatomen mono- oder disubstituierten Phenylrest substituiert sein kann, bedeuten.

2. Verfahren zur Herstellung von Verbindungen der Formel I nach Anspruch 1, dadurch gekennzeichnet, daß man ein Aminopyrrolin der Formel II

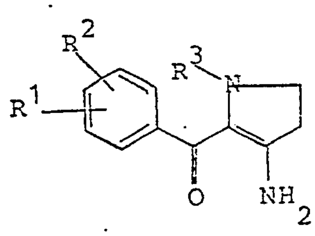

(II)

mit Hydrazin zu einer Verbindung der Formel I umsetzt, wobei $R^1$, $R^2$ und $R^3$ die in Anspruch 1 zu Formel I angegebenen Bedeutungen haben, und gegebenenfalls eine Verbindung der Formel I, in der $R^3$ Benzyl bedeutet, durch Abspalten der Benzylgruppe in eine Verbindung der Formel I überführt, in der $R^3$ Wasserstoff bedeutet.

3. Arzneimittel, gekennzeichnet durch einen Gehalt an einem Phenylpyrazolderivat der Formel I in Anspruch 1.

4. Phenylpyrazolderivats der Formel I in Anspruch zur Verwendung zur Hemmung der Magensäuresekretion sowie zur Prophylaxe und Therapie von Magenulcera.

5. Verwendung eines Phenylpyrazolderivats der Formel I in Anspruch 1 zur Herstellung eines Arzneimittels zur Hemmung der Magensäuresekretion sowie zur Prophylaxe und Therapie von Magenulcera.

6. Aminopyrroline der Formel II

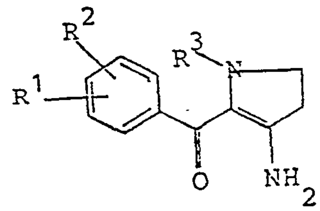

(II)

und ihre Salze mit physiologisch verträglichen Säuren, worin

$R^1$ und $R^2$ gleich oder verschieden sein können und Wasserstoff, Halogen, Alkyl oder Alkoxy mit 1 bis 4 Kohlenstoffatomen, Nitro oder Amino,

$R^3$ Wasserstoff, einen gesättigten oder ungesättigten, geradkettigen oder verzweigten aliphatischen Kohlenwasserstoffrest mit 1 bis 8 Kohlenstoffatomen, oder einen Cycloalkylrest mit 5 bis 8 Kohlenstoffatomen, wobei der Kohlenwasserstoffrest oder Cycloalkylrest seinerseits mit einem Phenylrest oder einem mit Halogen, Nitro, Amino oder einem Alkyl- oder Alkoxyrest mit 1 bis 4 Kohlenstoffatomen mono- oder disubstituierten Phenylrest substituiert sein kann, bedeuten.

7. Verfahren zur Herstellung der Aminopyrroline der Formel II in Anspruch 6, dadurch gekennzeichnet, daß man ein Aminoketon der Formel V

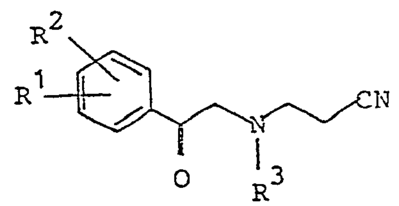

(V)

worin $R^1$, $R^2$ und $R^3$ die zu Formel II in Anspruch 6 angegebenen Bedeutungen haben, entweder mit einem Alkalialkoholat kondensiert, oder mit einer Lewis-Säure kondensiert und die so erhaltene Verbindung wäßrig alkalisch hydrolysiert.

**Patentansprüche** (für den Vertragsstaat AT)

1. Verfahren zur Herstellung von substituierten Phenylpyrazolderivaten der allgemeinen Formel I

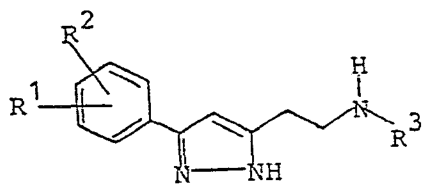

(I)

und ihrer Salze mit physiologisch verträglichen Säuren, worin

$R^1$ und $R^2$ gleich oder verschieden sein können und Wasserstoff, Halogen, Alkyl oder Alkoxy mit 1 bis 4 Kohlenstoffatomen, Nitro oder Amino,

$R^3$ Wasserstoff, einen gesättigten oder ungesättigten, geradkettigen oder verzweigten aliphatischen Kohlenwasserstoffrest mit 1 bis 8 Kohlenstoffatomen, oder einen Cycloalkylrest mit 5 bis 8 Kohlenstoffatomen, wobei der Kohlenwasserstoffrest oder Cycloalkylrest seinerseits mit einem Phenylrest oder einem mit Halogen, Nitro, Amino oder einem Alkyl- oder Alkoxyrest mit 1 bis 4 Kohlenstoffatomen mono- oder disubstituierten Phenylrest substituiert sein kann, bedeuten,
dadurch gekennzeichnet, daß man ein Aminopyrrolin der Formel II

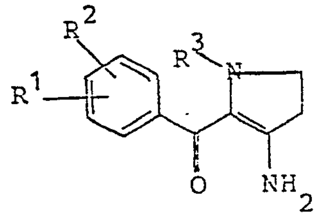

(II)

mit Hydrazin zu einer Verbindung der Formel I umsetzt, wobei $R^1$, $R^2$ und $R^3$ die zu Formel I angegebenen Bedeutungen haben, und gegebenenfalls eine Verbindung der Formel I, in der $R^3$ Benzyl

bedeutet, durch Abspalten der Benzylgruppe in eine Verbindung der Formel I überführt, in der $R^3$ Wasserstoff bedeutet.

2. Verfahren zur Herstellung der Aminopyrroline der Formel II

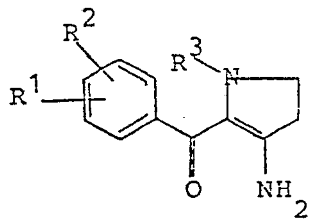

(II)

worin

$R^1$ und $R^2$ gleich oder verschieden sein können und Wasserstoff, Halogen, Alkyl oder Alkoxy mit 1 bis 4 Kohlenstoffatomen, Nitro oder Amino,

$R^3$ Wasserstoff, einen gesättigten oder ungesättigten, geradkettigen oder verzweigten aliphatischen Kohlenwasserstoffrest mit 1 bis 8 Kohlenstoffatomen, oder einen Cycloalkylrest mit 5 bis 8 Kohlenstoffatomen, wobei der Kohlenwasserstoffrest oder Cycloalkylrest seinerseits mit einem Phenylrest oder einem mit Halogen, Nitro, Amino oder einem Alkyl- oder Alkoxyrest mit 1 bis 4 Kohlenstoffatomen mono- oder disubstituierten Phenylrest substituiert sein kann, bedeuten,

dadurch gekennzeichnet, daß man ein Aminoketon der Formel V

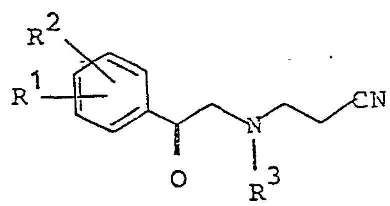

(V)

worin $R^1$, $R^2$ und $R^3$ die zu Formel II vorstehend angegebenen Bedeutungen haben, entweder mit einem Alkalialkoholat kondensiert, oder mit einer Lewis-Säure kondensiert und die so erhaltene Verbindung wäßrig alkalisch hydrolysiert.

**Claims** (for the Contracting States : BE, CH, DE, FR, GB, IT, LI, NL)

1. Substituted phenylpyrazole derivatives of the formula I

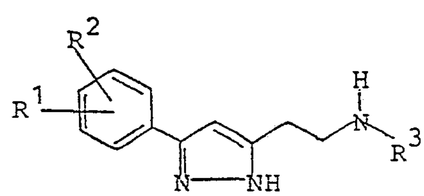

(I)

and their salts with physiologically acceptable acids, in which

$R^1$ and $R^2$, which may be identical or different, are hydrogen, halogen, alkyl or alkoxy having form 1 to 4 carbon atoms, nitro or amino,

$R^3$ is hydrogen, a saturated or unsaturated, linear or branched aliphatic hydrocarbon radical having from 1 to 8 carbon atoms, or a cycloalkyl radical having from 5 to 8 carbon atoms ; the hydrocarbon radical or cycloalkyl radical in turn may be substituted by a phenyl radical or a phenyl radical which may be mono- or disubstituted by halogen, nitro, amino or alkyl or alkoxy having from 1 to 4 carbon atoms.

2. A process for the preparation of compounds of the formula I as claimed in claim 1, which comprises reacting an aminopyrroline of the formula II

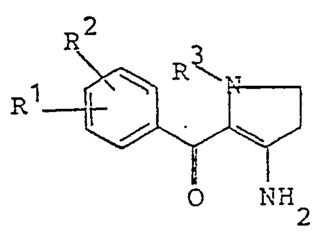

(II)

with hydrazine to yield a compound of the formula I, in which $R^1$ through $R^3$ are as defined in Claim 1 sub formula I and, if desired, converting a compound of the formula I in which $R^3$ is benzyl to a compound of the formula I in which $R^3$ is hydrogen by splitting off the benzyl group.

3. A pharmaceutical composition comprising as the active ingredient aphenylpyrazole derivative of the formula I as claimed in claim 1.

4. A phenylpyrazole derivative of the formula I as claimed in claim 1 for the use for inhibiting the secretion of gastric acid and for the prevention or therapy of gastric ulcers.

5. The use of a phenylpyrazole derivative of formula I as claimed in claim 1 for the manufacture of a pharmaceutical composition for the inhibition of the secretion of gastric acid and the prevention and therapy of gastric ulcers.

6. Aminopyrrolines of the formula II

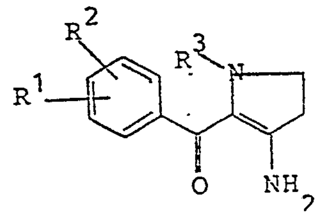

(II)

and their salts with physiologically acceptable acids, in which

$R^1$ and $R^2$, which may be identical or different, are hydrogen, halogen, alkyl or alkoxy having from 1 to 4 carbon atoms, nitro or amino,

$R^3$ is hydrogen, a saturated or unsaturated, linear or branched aliphatic hydrocarbon radical having from 1 to 8 carbon atoms, or a cycloalkyl radical having from 5 to 8 carbon atoms ; the hydrocarbon radical or cycloalkyl radical in turn may be substituted by a phenyl radical or a phenyl radical which may be mono- or disubstituted by halogen, nitro, amino or alkyl or alkoxy having from 1 to 4 carbon atoms.

7. A process for the preparation of the aminopyrrolines of the formula II as claimed in claim 6, which comprises condensing an aminoketone of the formula V

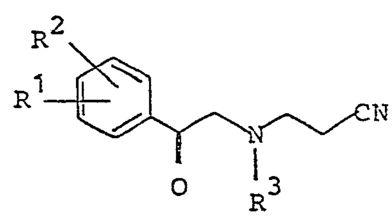

(V)

in which $R^1$ through $R^3$ are as defined for formula II as claimed in claim 6, either with an alkali metal alcoholate, or with a Lewis acid, and hydrolyzing the compound so obtained in an aqueous alkaline medium.

**Claims** (for the Contracting state AT)

1. A Process for the preparation of substituted phenylpyrazole derivatives of the formula I

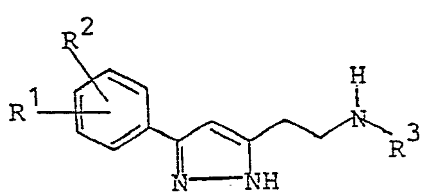

(I)

and of their salts with physiologically acceptable acids, in which

$R^1$ and $R^2$, which may be identical or different, are hydrogen, halogen, alkyl or alkoxy having from 1 to 4 carbon atoms, nitro or amino,

$R^3$ is hydrogen, a saturated or unsaturated, linear or branched aliphatic hydrocarbon radical having from 1 to 8 carbon atoms, or a cycloalkyl radical having from 5 to 8 carbon atoms ; the hydro-carbon radical or cycloalkyl radical in turn may be substituted by a phenyl radical or a phenyl radical which may be mono- or disubstituted by halogen, nitro, amino or alkyl or alkoxy having from 1 to 4 carbon atoms, which comprises reacting an aminopyrroline of the formula II

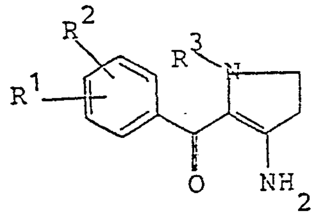

(II)

with hydrazine to yield a compound of the formula I, in which $R^1$ through $R^3$ are as defined above for formula I and, if desired, converting a compound of the formula I in which $R^3$ is benzyl to a compound of the formula I in which $R^3$ is hydrogen by splitting off the benzyl group.

2. A process for the preparation of aminopyrrolines of the formula II

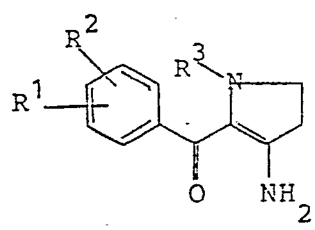

(II)

in which

$R^1$ and $R^2$, which may be identical or different, are hydrogen, halogen, alkyl or alkoxy having form 1 to 4 carbon atoms, nitro or amino,

$R^3$ is hydrogen, a saturated or unsaturated, linear or branched aliphatic hydrocarbon radical having from 1 to 8 carbon atoms, or a cycloalkyl radical having from 5 to 8 carbon atoms ; the hydrocarbon radical or cycloalkyl radical in turn may be substituted by a phenyl radical or a phenyl radical which may be mono- or disubstituted by halogen, nitro, amino or alkyl or alkoxy having from 1 to 4 carbon atoms which comprises condensing an amino ketone of the formula V

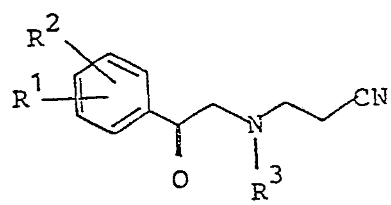

(V)

in which $R^1$ through $R^3$ are as defined for formula II, either with an alkali metal alcoholate, or with a Lewis acid, and hydrolyzing the compound so obtained in an aqueous alkaline medium.

**Revendications** (pour les Etats contractants : BE, CH, DE, FR, GB, IT, LI, NL)

1. Dérivés de phénylpyrazole substitués de formule générale I

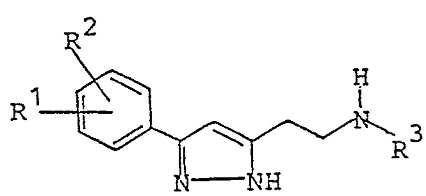

(I)

et leurs sels avec des acides physiologiquement acceptables, dans lesquels

$R^1$ et $R^2$ peuvent être indentiques ou différents et représentent l'hydrogène, un halogène, un groupe alcoyle ou alcoxy ayant de 1 à 4 atomes de carbone, nitro ou amino,

$R^3$ représente l'hydrogène, un radical hydrocarboné aliphatique, saturé ou insaturé, à chaîne droite ou ramifiée, ayant de 1 à 8 atomes de carbone, ou un radical cycloalcoyle ayant de 5 à 8 atomes de carbone, le radical hydrocarboné ou le radical cycloalcoyle pouvant de son côté être substitué avec un groupe phényle ou un groupe phényle mono- ou disubstitué avec un halogène, un groupe nitro, amino ou un groupe alcoyle ou alcoxy ayant de 1 à 4 atomes de carbone.

2. Procédé de préparation de composés de formule I selon la revendication 1, caractérisé en ce qu'on fait réagir une aminopyrroline de formule II

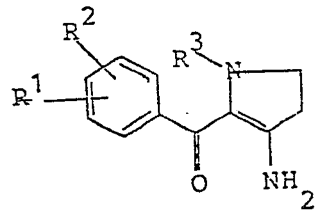

(II)

avec l'hydrazine pour obtenir un composé de formule I, $R^1$, $R^2$ et $R^3$ ayant alors les significations indiquées pour la formule I dans la revendication 1, et éventuellement par séparation du groupe benzyle, on convertit un composé de formule I, dans lequel $R^3$ représente un groupe benzyle, en un composé de formule I dans lequel $R^3$ représente l'hydrogène.

3. Médicament caractérisé par une teneur en un dérivé de phénylpyrazole de formule I selon la revendication 1.

4. Dérivé de phénylpyrazole dans formule I selon la revendication 1, pour utilisation dans l'inhibition de la sécrétion d'acide gastrique, ainsi que pour la prophylaxie et la thérapie des ulcères de l'estomac.

5. Utilisation d'un dérivé de phénylpyrazole de formule I selon la revendication 1, pour la préparation d'un médicament destiné à l'inhibition de la sécrétion d'acide gastrique, ainsi qu'à la prophylaxie et la thérapie des ulcères de l'estomac.

6. Aminopyrrolines de formule II

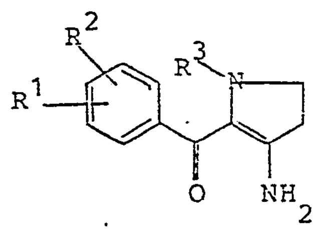

(II)

et leurs sels avec des acides physiologiquement acceptables, dans lesquels :

$R^1$ et $R^2$ peuvent être identiques ou différents et représentent l'hydrogène, un halogène, un groupe alcoyle ou alcoxy ayant de 1 à 4 atomes de carbone, nitro ou amino,

$R^3$ représente l'hydrogène, un radical hydrocarboné aliphatique, saturé ou insaturé, à chaîne droite ou ramifiée, ayant de 1 à 8 atomes de carbone, ou un radical cycloalcoyle ayant de 5 à 8 atomes de carbone, le radical hydrocarboné ou le radical cycloalcoyle pouvant être de son côté substitué avec un groupe phényle ou un groupe phényle mono- ou di-substitué avec un halogène, un groupe nitro, amino ou un groupe alcoyle ou alcoxy ayant de 1 à 4 atomes de carbone.

7. Procédé de préparation des aminopyrrolines de formule II selon la revendication 6, caractérisé en ce qu'on condense une aminocétone de formule V

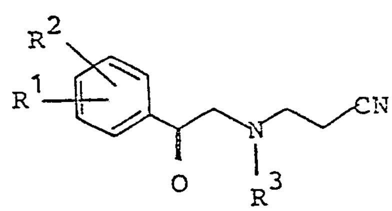

(V)

dans laquelle $R^1$, $R^2$ et $R^3$ ont les significations indiquées pour la formule II dans la revendication 6, soit avec un alcoolate alcalin, soit avec un acide de Lewis, et on hydrolyse le composé ainsi obtenu dans un milieu alcalin aqueux.

**Revendications** (pour l'Etat contractant AT)

1. Procédé de préparation de dérivés de phénylpyrazole substitués de formule générale I

(Voir formule p. 15)

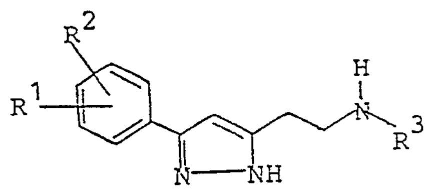

(I)

et leurs sels avec des acides physiologiquement acceptables, dans lesquels :

$R^1$ et $R^2$ peuvent être identiques ou différents et représentent l'hydrogène, un halogène, un groupe alcoyle ou alcoxy ayant de 1 à 4 atomes de carbone, nitro ou amino,

$R^3$ représente l'hydrogène, un radical hydrocarboné aliphatique, saturé ou insaturé, à chaîne droite ou ramifiée, ayant de 1 à 8 atomes de carbone, ou un radical cycloalcoyle ayant de 5 à 8 atomes de carbone, le radical hydrocarboné ou le radical cycloalcoyle pouvant être de son côté substitué avec un groupe phényle ou un groupe phényle mono- ou di-substitué avec un halogène, un groupe nitro, amino ou un groupe alcoyle ou alcoxy ayant de 1 à 4 atomes de carbone,

caractérisé en ce qu'on fait réagir une aminopyrroline de formule II

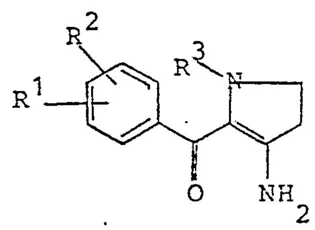

(II)

avec l'hydrazine pour obtenir un composé de formule I, $R^1$, $R^2$ et $R^3$ ayant les significations indiquées pour la formule I, et éventuellement par séparation du groupe benzyle, on convertit un composé de formule I dans lequel $R^3$ représente un groupe benzyle, en un composé de formule I dans lequel $R^3$ représente l'hydrogène.

2. Procédé de préparation d'aminopyrroline de formule II

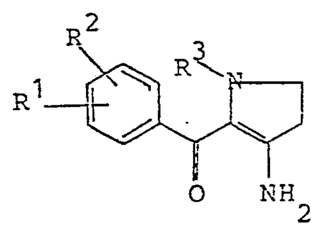

(II)

dans laquelle

$R^1$ et $R^2$ peuvent être identiques ou différents et représentent l'hydrogène, un halogène, un groupe alcoyle ou alcoxy ayant de 1 à 4 atomes de carbone, nitro ou amino,

$R^3$ représente l'hydrogène, un radical hydrocarboné aliphatique, saturé ou insaturé, à chaîne droite ou ramifiée, ayant de 1 à 8 atomes de carbone, ou un radical cycloalcoyle ayant de 5 à 6 atomes de carbone, le radical hydrocarboné ou le radical cycloalcoyle pouvant être de son côté substitué avec un groupe phényle ou un groupe phényle mono- ou disubstitué avec un halogène, un groupe nitro, amino ou un groupe alcoyle ou alcoxy ayant de 1 à 4 atomes de carbone,

caractérisé en ce qu'on condense une aminocétone de formule V

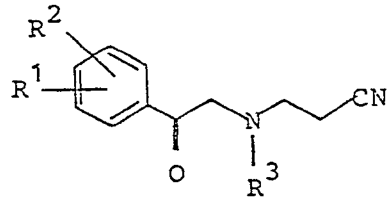

(V)

dans laquelle $R^1$, $R^2$ et $R^3$ ont les significations indiquées précédemment pour la formule II, soit avec un alcoolate alcalin, soit avec un acide de Lewis, et on hydrolyse le composé ainsi obtenu dans un milieu alcalin aqueux.